Europäisches Patentamt
European Patent Office
Office européen des brevets

(19)

(11) Publication number: **0 426 846 A1**

(12)

# EUROPEAN PATENT APPLICATION published in accordance with Art. 158(3) EPC

(21) Application number: 89907817.4

(22) Date of filing: 03.07.89

(86) International application number: PCT/JP89/00666

(87) International publication number: WO 90/00087 (11.01.90 90/02)

(51) Int. Cl.[5]: **B01J 31/02**, B01J 31/10, C07C 45/45, C07C 49/76, C07C 76/02, C07C 79/10, C08G 77/24, C08G 77/28, C08G 77/38

(30) Priority: 04.07.88 JP 166477/88

(43) Date of publication of application: 15.05.91 Bulletin 91/20

(84) Designated Contracting States: AT BE CH DE FR GB IT LI LU NL SE

(71) Applicant: MITSUI PETROCHEMICAL INDUSTRIES, LTD.
2-5, Kasumigaseki 3-chome Chiyoda-ku
Tokyo 100(JP)

(72) Inventor: TAKAHATA, Kazunori, Mitsui Petrochemical Industries, Ltd., 1-2, Waki 6-chome, Waki-cho
Kuga-gun, Yamaguchi 740(JP)
Inventor: KAYA, Hidenori, Mitsui Petrochemical Ind. , Ltd.
1-2, Waki 6-chome, Waki-cho, Kuga-gun
Yamaguchi 740(JP)
Inventor: TANIGUCHI, Katsuo, Mitsui Petrochemical Ind. Ltd.
1-2, Waki 6-chome, Waki-cho, Kuga-gun
Yamaguchi 740(JP)
Inventor: TAKAI, Toshihiro, Mitsui Petrochemical Ind. Ltd.
3, Chigusakaigan, Ichihara-shi
Chiba 299-01(JP)

(74) Representative: Myerscough, Philip Boyd et al
J.A.Kemp & Co. 14, South Square Gray's Inn
London, WC1R 5EU(GB)

## (54) FLUORINATED-AROMATIC SULFONIC ACID CATALYST, PROCESS FOR ITS PRODUCTION, AND ITS USE.

(57) The invention provides a fluorinated aromatic sulfonic acid catalyst in which sulfonic acid groups and aryl groups substituted by fluorine atoms or fluoroalkyl groups are bound to a matrix selected from the group consisting of polysiloxane and inorganic base material. This catalyst can be produced by introducing sulfonic acid groups and aryl groups substituted by fluorine atoms or fluoroalkyl groups into the above-described matrix. This catalyst is used in various acid-catalyzed reactions such as esterification, acylation, nitration, alkylation, etherification, etc., particularly acid-catalyzed reactions in the presence of water to show a high acid catalysis activity. In addition, since this catalyst is solid, its handling is so easy that recovery and reuse thereof is easy.

EP 0 426 846 A1

# FLUORINE-CONTAINING AROMATIC SULFONIC ACID CATALYST, PROCESS FOR THE PRODUCTION AND USE THEREOF

## Field of the Invention

The present invention relates to a catalyst for use for various acid catalyzed reactions, such as, esterification, acylation, nitration, alkylation, etherification and so on, especially a heterogeneous catalyst consisting of a matrix substance to which aryl groups having substituents of fluoride and sulfonic acid group are combined; as well as to a process for the production of such catalyst and use thereof.

## Background of the Invention

Among acid catalyzed reactions, there are many catalytic reactions in which water participates, such as, dehydration, hydration, alkylation, esterification, acylation, nitration and so on, which are all useful in industry. For the catalyst for such reactions, there have been employed in the practice various ones, such as, sulfuric acid, aluminum chloride, benzene sulfonic acid, silica-alumina, ion-exchange resins and so on.

For example, in the Japanese Patent Application Lay-Open No. 151106/1977, which discloses a process for the production of alcohols by hydrating lower olefines under the use of an ion-exchange resin of a type of fluorine-containing sulfonic acid as a heterogeneous catalyst, use of Nafion (trademark), a commercial product of a perfluorovinyl ether copolymer having sulfonic acid groups of E.I. du Pont de Nemours and Co., as catalyst for the production of secondary butyl alcohol by hydration of 1-butene and use of a resin product obtained by introducing fluorine into Amberlyst 15 (trademark), a commercial product of a sulfonated styrene/ divinyl benzene copolymer of Rohm and Haas Co., as catalyst for the production of sec.-butyl alcohohl by hydration of 1-butene are described respectively in Examples 2 and 3 thereof. In the USP No. 4,716,252 (corresponding to the Japanese Patent Application Lay-Open No. 33130/1987), there is disclosed a process for the production of bis-hydroxyphenyl-n-alkanes under the use of a catalyst consisting of a resin having combined thereto fluorine-containing aromatic sulfonic acid groups. However, the catalytic activity of such a resin is not recognizable to be sufficiently high.

Conventional acid catalysts suffer from objectionable problems. For example, sulfuric acid requires certain after-treatment, such as neutralization etc., silica-alumina is subject to decrease in the acid catalytic activity upon coexistence of water and aluminum chloride does not permit recovery to reuse it.

On the other hand, it is possible to produce hydroxyaryl aryl ketones very useful as intermediates for dyestuffs, high polymeric substances, stabilizers for synthetic resins and medicaments, by reacting aromatic carboxylic acids with phenols to obtain corresponding esters and subjecting these esters then to Fries rearrangement. Here, it has been practiced to use a catalyst, such as sulfuric acid or an ion-exchange resin, for the ester-forming reaction and, such as aluminum chloride, a polyphosphoric acid or so on, for the Fries rearrangement [Cf. Journal of Chemical Society C 650 (1971)] . Thus, this process may hardly be regarded as advantageous in industry, on account of necessity of annoying reaction procedures due to, for example, separate reaction steps for the esterification and for the Fries reaarangement, since the catalysts to be used are different for these process steps.

For these circumstances, the applicant had proposed employment of sulfonated polyphenylsiloxanes in the synthesis of hydroxyaryl aryl ketones, such as hydroxybenzophenone and so on, as the catalyst which permits to carry out the esterification and the Fries rearrangement within a single process step (Japanese Patent Application Serial No. 49415/1988). These catalysts remain, however, as being susceptible of further improvements in the points of catalytic activity etc.

There had further been disclosed a process for the production of acyl-substituted aromatic compounds from aromatic compounds and carboxylic acids, wherein layer-structured ion-exchangeable clays are used for the catalyst (Japanese Patent Application Lay-Open No.152636/1986).

This process suffers, however, from objectionable problem of lower catalytic activity.

An object of the present invention is to solve the above problems by providing an acid catalyst which is superior in the water-repellent property, exhibits a high acid catalytic activity even in the presence of water and allows easy separation from the reaction mixture.

Another object of the present invention is to provide a process for the production of such an acid catalyst and a method for applying such acid catalyst.

Description of the Invention

Thus, the present invention relates to a fluorine-containing aromatic sulfonic acid catalyst comprising a matrix substance selected from the group consisting of polysiloxanes and inorganic substances, to which arc combined substituted aryl groups represented by the general formula (I),

$$(X)_n - \underset{|}{Ar^1} - (SO_3H)_m \quad \cdots\cdots \quad (I)$$

wherein X denotes fluorine atom or a fluorine-containing $C_1$ - $C_{14}$ alkyl group which may or may not contain etheric oxygen atom, $A^1$ represents an aryl group, n is an integer of 1 - 5, with X being the same or different with each other for the case of n ≧ 2, and m is an integer of 1 - 3; as well as to a process for the production of such catalyst and use thereof.

The fluorine-containing aromatic sulfonic acid catalyst of the present invention comprises a matrix substance selected from the group consisting of polysiloxanes and inorganic substances, to which aryl groups each having substituents including fluoride and sulfonic acid group are combined.

For the inorganic substances, there may be enumerated, for example, compounds of metals, such as zirconium, aluminum, titanium and so on; clays such as bentonite, montmorillonite, mica and so on; and silica-alumina etc. One concrete example of the metal compound is zirconium phosphate. Among these substrates, zirconium compounds, micas and montmorillonites may preferably be employed.

For the aryl group represented by $Ar^1$ in the above formula (I), for example, phenyl, naphthyl, anthryl and so on may be enumerated.

For the fluorine-containing alkyl group represented by X in the above general formula (I), there may be exemplified concretely $-CF_3$, $-CF_2CF_3$, $-CF_2CF_2CF_3$, $-CF(CF_3)_2$, $-(CF_2)_3CF_3$, $-CH_2CF_3$, $-CH_2C_2F_5$, $-CH_2C_3F_7$, $-CH_2C_4F_9$, $-CH_2C_5F_{11}$, $-CH_2C_7F_{15}$, $-CH_2C_8F_{17}$, $-CH_2C_9F_{19}$, $-CH_2C_{10}F_{21}$, $-CH_2CH_2CF_3$, $-CH_2CH_2C_2F_5$, $-CH_2CH_2C_3F_7$, $-CH_2CH_2C_4F_9$, $-CH_2CH_2C_5F_{11}$, $-CH_2CH_2C_7F_{15}$, $-CH_2CH_2C_8F_{17}$, $-CH_2CH_2C_9F_{19}$, $-CH_2CH_2C_{10}F_{21}$, $-CH_2(CF_2)_2H$, $-CH_2\ (CF_2)_4H$, $-CH_2\ (CF_2)_6H$, $-CH_2(CF_2)_9H$, $-CH_2\ (CF_2)_{10}H$, $-CH(CF_3)_2$, $-CH_2CF_2CHFCF_3$, $-CH_2CF_2CHF(CF_2)_6H$, $-CH_2CF(CF_3)CHFCF\ (CF_3)_2$, $-CH_2CF(C_2F_5)CH(CF_3)_2$, $-CH_2C_6HF_{12}$, $-C_6HF_{12}$, $-CH_2C_{10}HF_{20}$, $-CH_2C_5F_{10}H$, $-(CH_2)_5OCF(CF_3)_2$, $-(CH_2)_{11}OCF(CF_3)_2$, $-CF_2(CF_2)_2OCF_3$, $-CH_2(CF_2)_2OC_2F_5$, $-CH_2(CF_2)_2OC_3F_7$, $-CH_2-CF_2-CFH-O-CF_2-CF(CF_3)-O-C_3F_7$, $-CF_2-CFH-O-CF_2-CF(CF_3)-O-C_3F_7$, $-CH_2-CF(CF_3)-O-CF_2-CF(CF_3)-O-C_3F_7$,

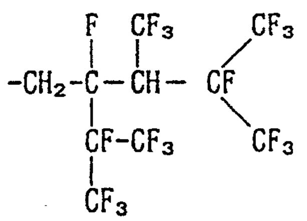 , 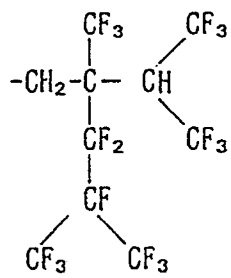 , 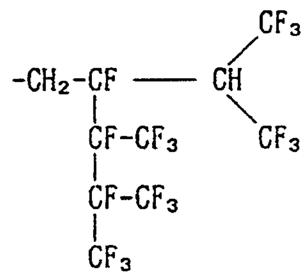

and so on. For the group for X, fluorine atom and $-CF_3$ are preferred.

The binding of the matrix substance with the aryl groups having substitents of fluoride and sulfonic acid group is realized by a chemical bond which may be different in accordance with each specific matrix substance employed and may be a covalent bond of carbon-silicon in case the matrix substance is a polysiloxane, may be a metal-carbon bond with highly covalent nature or with highly ionic nature due to the electronegativity of the metal in case the matrix substance is a metal compound. If the matrix substance is a clay, this binding may be realized by a metal-oxygen-carbon bond.

The aryl group having substituents of fluoride and sulfonic acid group may be combined with the matrix substance directly, while it is possible that the aryl group is combined with the matrix substance via, for example, an oxygen atom, a sulfur atom or a carbon atom. When the matrix substance has surface hydroxyl groups formed thereon, the aryl group can be combined therewith through oxygen atom by replacing the hydrogen atom of the surface hydroxyl group by the aryl group.

The fluorine-containing aromatic sulfonic acid catalyst of the present invention has a fluorine content in

the range from 0.1 to 50, preferably from 1 to 20 % by weight, and a sulfur content in the range from 0.1 to 30, preferably from 1 to 15 % by weight.

Due to the content of fluorine atom which has high electron attractivity, the acidity or acid strength of the sulfonic acid group of the fluorine-containing sulfonic acid catalyst of the present invention is increased and, in addition, it reveals superior water-repellent property as inherent to a fluorine-containing group. Facilitated by these properties, the fluorine-containing aromatic sulfonic acid catalyst of the present invention exhibits long-lasting high catalytic activity without being poisoned by water when employed for a catalytic reaction in an aqueous environment.

The prior art catalyst of (per)fluoroalkane sulfonic acid typically represented by $CF_3$-$SO_3H$ has a fatal defect of being susceptible of decomposition upon contact with water, due to its sensitiveness to moisture, so that it is, in general, difficult to use it for reactions in which water participates. In contrast thereto, the fluorine-containing aromatic sulfonic acid catalyst of the present invention is quite stable to moisture and will not decompose upon contact with water. Therefore, the catalyst according to the present invention can be employed for water-participating reactions and is also easy to handle, so that the validity in industry is far greater than the above mentioned catalyst based on (per)fluoroalkane sulfonic acid.

Aromatic compounds having substituents of fluoride and sulfonic acid group are, in general, soluble in various solvents, so that they have found their use as homogeneous catalyst and, therefore, separation of such catalysts is not easy. In contrast, the fluorine-containing aromatic sulfonic acid catalyst of the present invention exists as solid and is not soluble in ususal solvents, so that it is easy to handle and the recovery and reuse thereof can be realized easily.

Moreover, the fluorine-containing aromatic sulfonic acid catalyst of the present invntion has superior acid catalytic activity as compared with conventional solid catalyst employed heretofore as heterogeneous catalyst which is obtained by introducing into an ion-exchange resin fluoride radicals and sulfonic acid radicals.

For producing the fluorine-containing aromatic sulfonic acid catalyst according to the present invention, every technique can be employed, so long as a resulting product having the molecular structure in which the substituted aryl groups defined by the general formula (I) is combined to a matrix substance will be obtained, while it is recommended to pursue the procedures exemplified in the following production processes 1 to 5. In the descriptions of these processes 1 - 5, A and B represent each the pertinent reacting group and $Ar^1$ and X have the meanings as defined in the general formula (I).

Production Process 1 H1>

$$\text{Matrix-A} + \text{B-Ar}^1\text{-X} \xrightarrow{-\,\text{A}\cdot\text{B}} \text{Matrix-Ar}^1\text{-X} \xrightarrow{\text{Sulfonation}} \text{Objective catalyst}$$

This process comprises introducing a fluorine-containing aryl group into a matrix substance and sulfonating then the resulting product with a sulfonating agent.

Production Process 2 H1>

$$\text{Matrix-A} + \text{B-Ar}^1\text{-SO}_3\text{H} \xrightarrow{-\,\text{A}\cdot\text{B}} \text{Matrix-Ar}^1\text{-SO}_3\text{H} \xrightarrow{\text{Fluorination}} \text{Objective catalyst}$$

This process comprises introducing a sulfonic acid group-containing aryl group into a matrix substance and fluorinating then the resulting product with a fluorinating agent.

Production Process 3 H1>

$$\text{Matrix-Ar}^1 \xrightarrow{\text{Sulfonation}} \text{Matrix-Ar}^1\text{-SO}_3\text{H} \xrightarrow{\text{Fluorination}} \text{Objective catalyst}$$

This process comprises sulfonating the aryl group of a matrix substance into which aryl groups have already been introduced and fluorinating thereafter the so obtained sulfonated product using a fluorinating agent.

Production Process 4 H1>

$$\text{Matrix-}Ar^1 \xrightarrow{\text{Fluorination}} \text{Matrix-}Ar^1\text{-}X \xrightarrow{\text{Sulfonation}} \text{Objective catalyst}$$

This process comprises fluorinating the aryl group of a matrix substance into which aryl groups have already been introduced and sulfonating thereafter the so obtained fluorinated product using a sulfonating agent.

Production Process 5 H1>

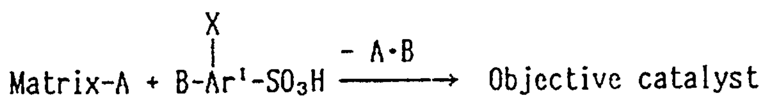


This process comprises introducing aryl groups each having fluoride and sulfonic acid groups into a matrix substance

In the above processes, the fluorinating agent may be known one, such as fluorinated xenon, gaseous fluorine or so on. As the sulfonating agent, also known one may be employed, for example, chlorosulfuric acid, concentrated sulfuric acid and so on.

The fluorine-containing aromatic sulfonic acid catalyst obtained as above is present usually in a form of powder or particles and can be used for catalyst as such or, if necessary, after processing into a shaped product, such as spheres, cylinders, honeycomb profils and so on, so that it is easy in handling and recovery by, for example, filtration etc. for reusing it.

The fluorine-containing aromatic sulfonic acid catalyst of the present invention can be used for various acid catalysis reactions, such as, esterification, acylation, alkylation, nitration, etherification and so on. Among the acid catalysis reactions, those in which water participates are especially recommended in applying the catalyst according to the present invention for a more efficient utilization of the characteristic nature of the catalyst.

⟨⟨Fluorine-containing Sulfonated Polyphenylsiloxane⟩⟩

Now, the description is directed to fluorine-containing sulfonated polyphenylsiloxane in which a polysiloxane is employed for the matrix substance and $Ar^1$ of the general formula (I) is a substituted phenyl group.

A fluorine-containing sulfonated polysiloxane includes a repeating unit expressed by the following general formula (V-a):

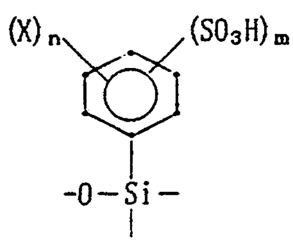

···· (V-a)

in which X, n and m have the same meanings as in the general formula (I).

A fluorine-containing sulfonated polysiloxane according to the present invention may include beside the

repeating unit expressed by the general formula (V-a) further repeating units expressed by the general formulae (V-b) to (V-d) respectively each within an extent up to which the characteristic nature of high acid catalysis activity of the catalyst according to the invention will not be lost:

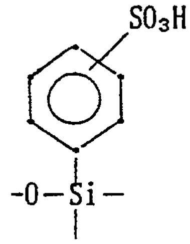 (V-b) 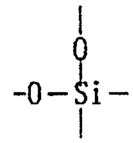 (V-c) 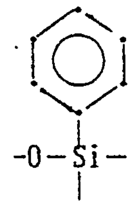 (V-d)

The fluorine-containing sulfonated polyphenylsiloxanes composed of the above repeating units may have a fluorine content usually in the range from 0.1 to 50 % by weight, a sulfur content usually in the range from 0.1 to 30 % by weight, a silicon content usually in the range from 10 to 90 % by weight and a carbon content usually in the range from 1 to 50 % by weight.

In the production of fluorine-containing sulfonated polyphenylsiloxanes according to the present invention, the fluorination and the sulfonation of the polyphenylsiloxane may be effected simultaneously, while it is preferable to conduct them each in a separate process step, since the adequate reaction condition is different for them, wherein either of them can be carried out in advance to the other.

Thus, a first concrete process for producing the fluorine-containing sulfonated polyphenylsiloxane from a polyphenylsiloxane may comprise fluorinating the polyphenylsiloxane with a fluorine-containing compound and sulfonating then the so fluorinated product with a sulfonating agent (as corresponding to the above production process 4). A second concrete process may comprise fluorinating, using a fluorinating agent, a polyphenylsiloxane which has been sulfonated beforehand by a sulfonating agent, to obtain the fluorine-containing sulfonated polyphenylsiloxane (as corresponding to the above production process 3).

In the above first and the second process for the production of fluorine-containing sulfonated polyphenylsiloxanes, the starting polyphenylsiloxane may be any polyphenylsiloxane containing phenyl groups and having a molecular structure composed of at least the repeating units represented by the general formulae (VI-a) and (VI-b) respectively:

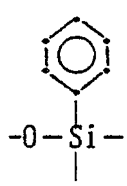 (VI-a) 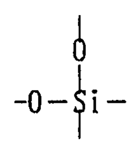 (VI-b)

Here, the mole ratio of the repeating unit of general formula (VI-a) to that of the general formula (VI-b) may be in the range from 9:1 to 1:9. The phenyl group of the formula (VI-a) can be combined to the silicon atom either directly or under intermediation by, such as, an alkylene group containing 1 - 6 carbon atoms.

Polyphenylsiloxanes having the above molecular structure can be produced, for example, by the procedures as given below:

A phenyltrialkoxysilane such as $C_6H_5Si(OC_2H_5)_3$ and a tetraalkoxysilane such as $Si(OC_2H_5)_4$ are mixed as the starting materials in a mole ratio in the range from 1:9 to 7:3 and, to the so obtained mixture, an alcohol such as ethanol and a dilute acid such as about 0.1 N dilute hydrochloric acid are added and the reaction is carried out at a temperature near the boiling point of the alcohol employed, for example, at about 80 °C in case ethanol is employed. After the reaction has been completed, the alcohol is distilled off and the reaction product is dissolved in a 0.5 - 2 times weight of an organic solvent such as hexane and thereto are then added 1 - 100 times weight, preferably 5 - 20 times weight of water under vigorous agitation, whereupon a 0.1 - 1 time weight of concentrated aqueous ammonia is added thereto and agitation is further continued for 1 - 10 hours. Then, the white pulverous solid product is separated by filtration or centrifugation and is washed with, for example, water, whereupon the separated cake is subjected to drying under a pressure of from vacuum to ordinary pressure at a temperature from room temperature to 200 °C to obtain the objective polyphenylsiloxane.

The fluorine-containing sulfonated polyphenylsiloxane can be obtained in the above first process by fluorinating the polyphenylsiloxane prepared as above using a fluorine-containing compound and sulfonating the so fluorinated product using a sulfonating agent. In the second process, it is produced by fluorinating a sulfonated polyphenylsiloxane using a fluorine-containing compound. Thus, in the above first process, the polyphenylsiloxane obtained above is employed as the starting material and is introduced into an organic solvent and the fluorination is carried out using a fluorinating agent at a temperature of 0 - 50 °C for 1 - 5 hours to obtain the objective fluorine-containing polyphenylsiloxane. As the fluorinating agent, for example, fluorinated xenon, gaseous fluorine and so on may be employed either solely or in a mixture of two or more of such fluorinating compounds.

For the solvent for conducting these reactions, every organic solvent inert to the reaction according to the present invention, such as, carbon tetrachloride, chloroform and so on, may be employed either solely or in a mixture of two or more of them.

The thus obtained fluorine-containing polyphenylsiloxane is subjected to sulfonation by adding thereto a sulfonating agent and reacting at a temperature of 0 - 80 °C for 2 - 8 hours to obtain the objective fluorine-containing sulfonated polyphenylsiloxane. Here, as the sulfonating agent, for example, chlorosulfuric acid, concentrated sulfuric acid and so on may be employed either solely or in a mixture of two or more of them.

In the second process for producing the fluorine-containing polyphenylsiloxane, the polyphenylsiloxane prepared as above is employed as the starting material and the sulfonation is effected as in the above first process, whereupon the so obtained sulfonated polyphenylsiloxane is fluorinated in a similar manner as in the above first process to obtain the objective fluorine-containing sulfonated polyphenylsiloxane.

Alternatively, there may be employed other processes, such as those which comprise producing first a fluorine-containing polyphenylsiloxane and subjecting this to sulfonation using a sulfonating agent (as corresponding to the above production process 1) and which comprise producing first polyphenylsiloxane in which sulfonic acid groups have been introduced and then subjecting this to fluorination using a fluorine-containing compound (as corresponding to the above production process 2), in order to produce the fluorine-containing sulfonated polyphenylsiloxane.

The fluorine-containing polyphenylsiloxane can be obtained, for example, by the procedures as given below:

Thus, a fluorine-containing phenyltrialkoxysilane is first produced by reacting a halogenated trialkoxysilane, such as chlorotriethoxysilane with a fluorine-containing phenyl metal halide, such as p-fluorophenyl magnesium bromide or 3,5-bis(trifluoromethyl)phenyl magnesium bromide in an organic solvent, such as ether, with cooling. Then, the so obtained fluorine-containing phenyltrialkoxysilane is used as the starting material together with a tetraalkoxysilane, such as tetraethoxysilane, and these starting materials are treated in the same manner as in the above procedures for producing the polyphenylsiloxane, whereby a contemplated fluorine-containing polyphenylsiloxane can be obtained.

for producing the fluorine-containing sulfonated polyphenylsiloxane from the thus obtained fluorine-containing polyphenylsiloxane, similar procedures for the sulfonation as in the above first process may be employed.

The fluorine-containing sulfonated polyphenylsiloxane catalyst according to the present invention exhibits a very high catalytic activity and can be used as the catalyst in the process for producing hydroxyaryl aryl ketones from phenols expressed by the general formula (II) as given below and aromatic carboxylic acids as represented by the general formula (III) as given afterwards through esterification and Fries rearrangement, as the catalyst in the process for the production of acyl-substituted aromatic compounds by reacting an aromatic compound expressed by the genral formula (IV) given afterwards with a carboxylic acid and as the catalyst in the production of nitrobenzenes by nitrating benzenes using nitrating agent and so on.

Now, the description is directed to the process for the production of hydroxyaryl aryl ketones using the fluorine-containing sulfonated polyphenylsiloxane obtained according to the present invention.

The starting phenols to be employed in the process for the production of hydroxyaryl aryl ketones may be represented by the following general formula (II):

$$Ar^2\text{-}(OH)_m \quad \cdots\cdots \quad (II)$$

in which $Ar^2$ represents a radical of an aromatic compound and m is an integer of at least one.

As the radical of aromatic compound represented by $Ar^2$ in the above formula, there may be enumerated, for example, substituted or unsubstituted rings of benzene, naphthalene, anthracene and tetralin. For the substituents for the radical $Ar^2$, there may be enumerated, for example, halogen atoms,

nitro, alkyl, alkoxy, phenoxy, amino, aryl, alkoxycarbonyl, phenoxycarbonyl and carboxyl. m may preferably be 1 - 2. Concrete examples of such phenols include hydroxybenzene (i.e. phenol), o-, m- and p-cresols, catechol, resorcinol, hydroquinone, pyrogallol, o-, m- and p-chlorophenols, 3,5-xylenol, 2,6-xylenol, m- and p-aminophenols, o-, m- and p-methoxyphenols, α- and β-naphthols, 2,6-naphthalenediol, α- and β-anthrols, dihydroxydiphenyl ether, bisphenol A and biphenol. These phenols may be employed either solely or in a mixture of two or more of them.

The aromatic carboxylic acids to be employed for the production of hydroxyaryl aryl ketones may be represented by the general formula (III):

$$Ar^3-(COOH)_n \qquad \cdots\cdot (III)$$

in which $Ar^3$ denotes a radical of an aromatic compound and n is an integer of at least one.

As the radical of aromatic compound represented by $Ar^3$ in the above formula, there may be enumerated, for example, substituted or unsubstituted rings of benzene, naphthalene, anthracene and tetralin. For the substituents for the radical $Ar^3$, there may be enumerated, for example, halogen atoms, nitro, alkyl, alkoxy, hydroxy, phenoxy, amino, aryl, alkoxycarbonyl and phenoxycarbonyl. n may preferably be 1 - 2. Examples of these aromatic carboxylic acids include benzoic acid, hydroxybenzoic acid, p-aminobenzoic acid, 2,4-dichlorobenzoic acid, p-tert-butylbenzoic acid, benzoylbenzoic acid, salicylic acid, protocatechuic acid, phthalic acid, isophthalic acid, terephthalic acid, biphenyldicarboxylic acid, naphthoic acid, β -naphthoic acid, naphthalenedicarboxylic acid and anthranilic acid as well as the possible acid anhydrides and salts of these acids. These aromatic carboxylic acids can be employed either solely or in a mixture of two or more of them.

While the reaction of the phenols with the aromatic carboxylic acids as above may be carried out without employing solvent, it is preferable to conduct the reaction in an organic solvent as the reaction medium. Here, it is furthermore preferable to employ an organic solvent which is immiscible with water and nevertheless is capable of forming an azeotrope with water.

Examples of such an organic solvent include aromatic compounds, such as, benzene, toluene, xylene and mesitylene; aliphatic compounds, such as, hexane, octane, cyclohexane, decalin and decane; and aromatic halogenated compounds, such as, chlorobenzene, dichlorobenzene and bromobenzene. These organic solvents may be employed either solely or in a mixture of two or more of them.

While it is possible to react a phenol with a carboxylic acid in a mole ratio within the range from 1 to 1,000 moles of the aromatic carboxylic acid per 100 moles of the phenol in producing the hydroxyaryl aryl ketones, it is especially advantageous to effect the reaction of these reactants in such a mole ratio that the phenol is present in excess of the aromatic carboxylic acid. It is especially preferable to carry out the reaction of the aromatic carboxylic acid and the phenol in a mole ratio of 10 - 90 moles of the aromatic carboxylic acid per 100 moles of the phenol.

The amount of the fluorine-containing sulfonated polyphenylsiloxane catalyst to be employed in the production of hydroxyaryl aryl ketones may, in general, be in the range from 0.1 to 50, preferably from 1 to 20 parts by weight per 100 parts by weight of the sum of the phenol and the aromatic carboxylic acid employed, while some deviation may be possible.

The temperature of the reaction of the phenol with the aromatic carboxylic acid may usually be in the range from 100 to 400 °C, in particular, in the range from 150 to 350°C.

The reaction duration may adequately be settled on account of the reaction temperature, the mode of the reaction and so on. For the case of conducting the reaction in a liquid phase, it is preferable to settle the reaction duration within the range from 1 to 20 hours.

The above reaction can be carried out either under a reduced pressure, ordinary pressure or a pressurized condition either in a gaseous or liquid phase. When carrying out the reaction in a liquid phase, ordinary technique, such as in a batch wise or half batch wise reaction or a continuous rection using a tubular reactor may be employed. When the reaction is carried out in gaseous phase, a fixed bed or a fluidized bed technique may be employed.

It is preferable in the above production process to conduct the reaction while removing the reaction water formed during the reaction out of the reaction system continuously. Here, it is particularly preferable to remove the reaction water from the reaction system as an azeotropic mixture with the solvent or with the starting phenol compound.

Upon carrying out the reaction under such conditions, an ester compound is formed first by the reaction of the phenol with the aromatic carboxylic acid. By further proceeding the reaction, the thus formed ester compound will be converted into a corresponding ketone compound, namely the corresponding hydroxyaryl

aryl keton represented by the following general formula (VII) due to Fries rearrangement:

$$HO-Ar^2-\underset{\underset{O}{\|}}{C}-Ar^3 \qquad \cdots\cdot (VII)$$

in which $Ar^2$ and $Ar^3$ have the same meanings as those in the general formulae (II) and (III).

Concrete examples of the hydroxyaryl aryl ketones to be obtained by the above production process include benzophenones, such as, 4,4'-dihydroxybenzophenone 2,4-dihydroxybenzophenone, 2,5-dihydroxybenzophenone, 2-hydroxybenzophenone, 4-hydroxybenzophenone, 4-hydroxy-4'-chlorobenzophenone, 2,4,4'-trihydroxybenzophenone, 2,3,4-trihydroxybenzophenone, 2,3,4,4'-tetrahydroxybenzophenone, 2-methyl-4-hydroxybenzophenone, 2-hydroxy-5-methylbenzophenone,2-hydroxy-4'-methoxybenzophenone, 4-hydroxy-4'-methoxybenzophenone; and phenylnaphthylketones, such as, 4-hydroxyphenyl-1-naphthylketone, 4-hydroxyphenyl-2-naphthylketone, 4-hydroxyphenyl-2-(6-hydroxynaphthyl)ketone 6,6'-dihydroxy-2,2'-dinaphthylketone and so on.

The following reaction formulae (VIII) and (IX) may typically exemplify the reaction in the above production processes:

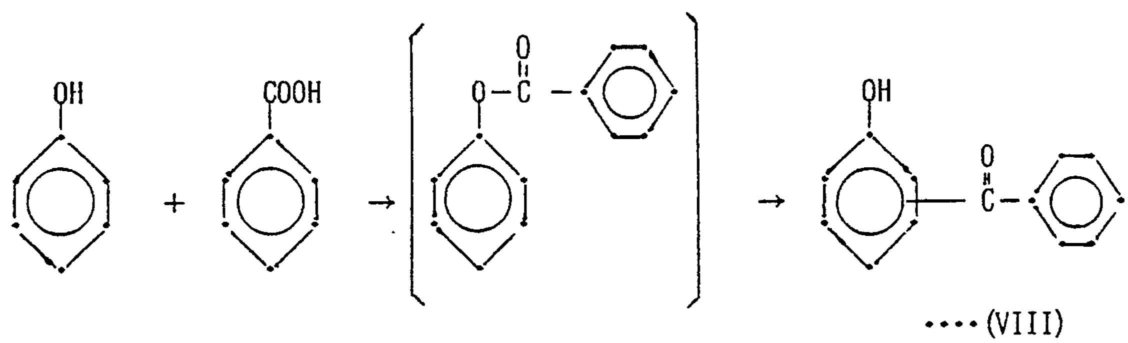

···· (VIII)

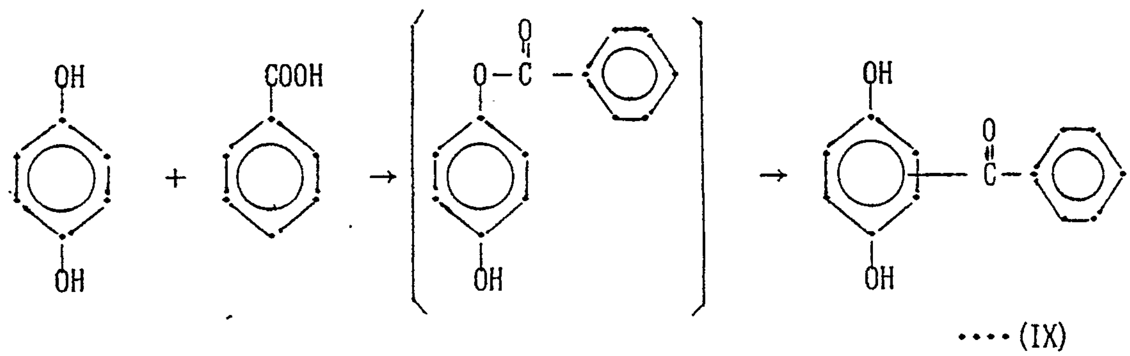

···· (IX)

In the reaction of the former formula, hydroxybenzophenones are formed from phenol and benzoic acid, while in the reaction of the latter formula, dihydroxybenzophenones are formed from hydroquinone and benzoic acid.

In the above process, the phenols and the aromatic carboxylic acids can be reacted in a very high conversion and at a very high selectivity to yield the corresponding hydroxyaryl aryl ketones, such as hydroxybenzophenone and so on, since the catalytic activity of the fluorine-containing sulfonated polyphenylsiloxane according to the present invention is quite high.

Another possible reaction for which the fluorine-containing sulfonated polyphenylsiloxane to be obtained according to the present invention can be employed is nitration of benzenes carried out in an organic solvent or without using such a solvent in the presence of a nitrating agent, such as nitric acid, nitrogen dioxide or so on, to produce nitrobenzenes.

Benzenes to be used in the above catalytic nitration may have substituents impervious to nitration and may be benzene, toluene, naphthalene, methylnaphthalene, anthracene and so on.

The amount of the fluorine-containing sulfonated polyphenylsiloxane catalyst to be employed in the production of nitrobenzenes may preferably be those which will satisfy a LHSV value in the range of 0.1 - 20 $hr^{-1}$, preferably in the range of 1 - 10 $hr^{-1}$, while some deviations may be possible.

A concrete example of such a reaction may be represented by the following reaction formula (X):

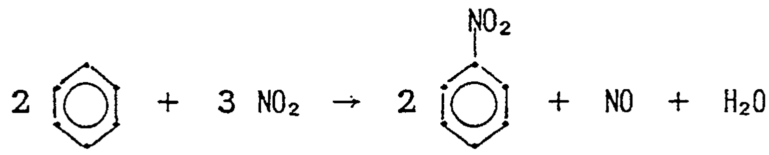

···· (X)

This reaction employs benzene as the starting substance and is effected using nitrogen dioxide as the nitrating agent to produce nitrobenzene.

In the following, production of an acyl-substituted aromatic compound by reacting an aromatic compound with a carboxylic compound using the fluorine-containing sulfonated polyphenylsiloxane produced according to the present invention is explained.

The aromatic compound to be employed in the production of the acyl-substituted aromatic compound is that represented by the general formula (IV):

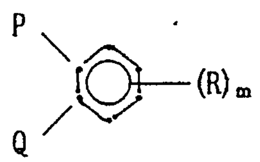

···· (IV)

wherein R represents hydrogen atom, an alkyl group, an alkoxy group or a halogen atom, m is an integer of 1 - 3 and P and Q denote both hydrogen atom or either of them is phenyl group and the other is hydrogen atom or they represent each a hydrocarbon group combining with each other to build up a substituted or unsubstituted naphthalene or tetralin ring.

Concrete examples of such an aromatic compound represented by the above general formula (IV) include alkylbenzenes, such as, benzene, toluene, o-, m- and p-xylene, mesitylene, pseudocumene, 1,2,4,5-tetramethylbenzene, ethylbenzene, n-propylbenzene, isopropylbenzene, butylbenzene, diethylbenzene and diisopropylbenzene; alkoxybenzenes, such as, anisole, methylmethoxybenzene, ethylmethoxybenzene, ethoxybenzene, methylethoxybenzene and propoxybenzene; halogenated benzenes, such as, chlorobenzene, methylchlorobenzene, methoxychlorobenzene, bromobenzene, methylbromobenzene, iodobenzene and fluorobenzene; biphenyl; naphthalenes, such as, naphthalene, α-methylnaphthalene, β-methylnaphthalene, 2,6-dimethylnaphthalene, methylchloronaphthalene and methoxynaphthalene; and tetralin. Among them, toluene, o-, m- and p-xylenes, mesitylene, anisole, naphthalene, methylnaphthalene, dimethylnaphthalene and methoxynaphthalene are preferable because of high reaction yield.

Examples of carboxylic acid to be employed in the process for the production of acyl-substituted aromatic compounds according to the present invention include aliphatic carboxylic acids, such as, formic acid, acetic caid, propionic acid, stearic acid, lauric acid, acrylic acid, methacrylic acid, oxalic acid, succinic acid and maleic acid; substituted aliphatic carboxilic acids having substituent of substituted or unsubstituted aromatic hydrocarbon residue, such as, phenylacetic acid and cinnamic acid; aromatic carboxylic acids, such as, benzoic acid, toluic acid, ethylbenzoic acid, phenylbenzoic acid, hydroxybenzoic acid, methoxybenzoic acid, arylbenzoic acid, chlorobenzoic acid, nitrobenzoic acid, phthalic acid, isophthalic acid, terephthalic acid, naphthoic acid and naphthalenedicarboxylic acid; acid anhydrides, such as, acetic anhydride, propionic anhydride, benzoic anhydride and phthalic anhydride; acid halogenides, such as, acetyl chloride, benzoyl chloride and so on; and ketocarboxylic acids, such as pyruvic acid and so on.

The amount of the fluorine-containing sulfonated polyphenylsiloxane to be employed in the production of acyl-substituted aromatic compounds may, in general, be in the range from 0.1 to 100, preferably from 1 to 30 parts by weight per 100 parts by weight of the sum of the aromatic compound and carboxylic acid used as the starting materials, while some deviations may be possible.

In the acylation, the aromatic compound as exemplified above is reacted with the carboxylic acid as given above in the presence of the fluorine-containing sulfonated polyphenylsiloxane catalyst to obtain corresponding acyl-substituted compound. Concrete examples of the acyl-substituted aromatic compound

thus obtained include alkyl phenyl ketones, such as, benzaldehyde, acetophenone, ethyl phenyl ketone, n-propyl phenyl ketone, propyl phenyl ketone and butyl phenyl ketone; alkyl aryl ketones, such as, 2-methylbenzaldehyde, 4-methylacetophenone, 2,4-dimethylacetophenone, 2,4,6-trimethylacetophenone, 2-ethylacetophenone, 4-methoxyacetophenone, 4-ethoxyacetophenone, 2,4-dimethylphenyl ethyl ketone, 2,4,6-trimethylphenyl ethyl ketone,2,4,6-trimethylphenyl propyl ketone and 4-methoxyphenyl butyl ketone; diaryl ketones, such as, benzophenone, 2-methylbenzophenone, 4-ethylbenzophenone, 2,4-dimethylbenzophenone, 3,4-dimethylbenzophenone, 2,5-dimethylbenzophenone, 2,4,6-trimethylbenzophenone, 2,4-dimethyl-4'-methylbenzophenone, 2,4,6-trimethyl-4'-methylbenzophenone, 2,4-diethyl-4'-methylbenzophenone, 2,4-dimethyl-4'-methoxybenzophenone, 4-methoxybenzophenone, 4-methoxy-4'-methylbenzophenone and 4,4'-dimethoxybenzophenone; alkyl naphthyl ketones, such as, 1-naphthyl methyl ketone, 2-naphthyl methyl ketone, 1-naphthyl ethyl ketone, 1-naphthyl propyl ketone, 1-(6-methylnaphthyl) methyl ketone, 2-(1-methoxynaphthyl) methyl ketone and 2-(1-methoxynaphthyl) ethyl ketone; phenyl naphthyl ketones, such as, phenyl-1-naphthyl ketone, phenyl-2-naphthyl ketone, phenyl-1-(2-methylnaphthyl) ketone, phenyl-1-(6-methylnaphthyl) ketone, phenyl-1-(2,6-dimethylnaphthyl) ketone, phenyl-1-(6-ethylnaphthyl) ketone, phenyl-1-(2-methyl-4-ethylnaphthyl) ketone, phenyl-1-(6-propylnaphthyl) ketone, phenyl-2-(2-methylnaphthyl) ketone, phenyl-2-(6-methylnaphthyl) ketone and phenyl-2-(1-methoxynaphthyl) ketone; aryl naphthyl ketones, such as, tolyl-1-naphthyl ketone, tolyl-2-naphthyl ketone, 2,4-dimethylphenyl-1'-naphthyl ketone, 2,4-dimethylphenyl-2'-naphthyl ketone, 2,4,6-trimethylphenyl-1'-naphthyl ketone, 2,4,6-trimethylphenyl-2'-naphthyl ketone, 2,4-dimethylphenyl-1'-(4'-methylnaphthyl) ketone, 2,4,6-trimethylphenyl-l'-(6'-methylnaphthyl)ketone, 4-methoxyphenyl-1'-naphthyl ketone, 4-methoxyphenyl-2-naphthyl ketone and 4-methoxyphenyl-2'-(6'-methoxynaphthyl) ketone; dinaphthyl ketones, such as, 1,1'-dinaphthyl ketone, 1,2'-dinaphthyl ketone, 2,2'-dinaphthyl ketone, 1-(2-methylnaphthyl)-1'-naphthyl ketone, 1-(5-methylnaphthyl)-1'-naphthyl ketone, 1-(6-methylnaphthyl)-1'-naphthyl ketone, 1-(4,6-dimethylnaphthyl)-1'-naphthyl ketone, 1-(5-ethylnaphthyl)-1'-naphthyl ketone, 1-naphthyl-1'-(6'-methylnaphthyl) ketone, 1-(4-methylnaphthyl)-1'-(6'-methylnaphthyl) ketone, 1-(4-methylnaphthyl)-2'-naphthyl ketone, 1-(6-propylnaphthyl)-2'-naphthyl ketone, 1-naphthyl-2'-(6'-methylnaphthyl) ketone, 1-(6-methylnaphthyl)-2'-(4'-methylnaphthyl) ketone, 2-(6-methylnaphthyl)-2'-(4',7'-dimethylnaphthyl) ketone, 2-(1-methoxynaphthyl)-1'-naphthyl ketone, 2-(1-methoxynaphthyl)-1'-(6'-methylnaphthyl) ketone, 2-(1-methoxynaphthyl)-1'-(4'-methoxynaphthyl) ketone and so on.

Among the above acyl-substituted aromatic compounds, especially the diaryl ketones of methylbenzophenone, dimethylbenzophenone, trimethylbenzophenone and methoxybenzophenone; the alkyl aryl ketones of methylacetophenone and dimethylacetophenone; the phenyl naphthyl ketones of phenyl naphthyl ketone and phenyl methoxynaphthyl ketone; the aryl naphthyl ketones of methoxyphenyl naphthyl ketone and phenyl methoxynaphthyl keton; and the alkyl naphthyl ketone of methoxynaphthyl methyl ketone are preferable because of high reaction yield.

While the above reaction may be carried out either in liquid phase and in gaseous phase, it is more preferable to carry out in liquid phase because of the favorable reaction yield.

The reaction temperature in the acylation may usually be in the range from room temperature to 350 °C, preferably in the range from 100 to 250 °Ç. The reaction may be carried out either in liquid phase or in gaseous phase on requirement. The reaction duration may voluntarily be chosen, while it may be sufficient usually to choose a reaction time of 0.1 - 20 hours, especially for liquid phase reaction.

⟨⟨Fluorine-containing Aromatic Sulfonic Acid/Zirconium Compound Catalyst⟩⟩

Now, explanations for the fluorine-containing aromatic sulfonic acid/zirconium compound catalyst in which the matrix substance is a zirconium compound are given.

As the zirconium compounds to be employed for the matrix substance, zirconium phosphate etc. may be exemplified.

For producing the fluorine-containing aromatic sulfonic acid/ zirconium compound catalyst, it is able to employ a method of introducing an aromatic group having substituents of fluoride and sulfonic acid group into the zirconium compound (corresponding to the production process 5 explained previously).

Now, preparation of the catalyst in which the matrix substance is a zirconium substance is explained by reciting concrete procedures. To an aqeous phosphoric acid solution of 0.1 - 10 M, an aqueous solution of a zirconium salt, such as, zirconium oxychloride, in a concentration of 0.1 - 5 M is added and the reaction is effected at a lower temperature (not higher than room -temperature) for 1 - 30 hours. After completion of the reaction, the solid reaction product is filtered off and is then reslurried in an aqueous solution of phosphoric acid of a concentration of 1 - 10 % by weight and this slurry is conditioned at a temperature from room temperature to 80 °C for 0.5 - 5 hours. After this treatment, the solid matter is separated and dried at a

temperature of about 100 °C. To this dried product, an aqueous solution of phosphoric acid of a concentration of 5 - 25 M is added and the mixture is heated at a temperature of 140 - 200°C for 5 - 20 hours to obtain crystalline zirconium phosphate. 1 part by weight of the so obtained crystalline zirconium phosphate and 1 - 10 parts by weight of an aromatic compound having substituents of fluoride and sulfonic acid group, such as, chloromethylfluorobenzenesulfonic acid, are introduced into an adequate amount of an organic solvent and are caused to react in the presence of an organic or inorganic base at a temperature of 0 - 100°C, whereby a fluorine-containing aromatic sulfonic acid/zirconium phosphate catalyst, namely, a compound in which the aromatic group having substituents of fluoride and sulfonic acid group is combined to zirconium phosphate, can be obtained.

The fluorine-containing aromatic sulfonic acid/zirconium compound catalyst produced in this manner can be utilized for the production of hydroxyaryl aryl ketones from phenol compounds expressed by the general formula (II) and aromatic carboxylic compounds represented by the general formula (III) through esterification and Fries rearrangement, for the production of acyl-substituted aromatic compounds by reacting the aromatic compounds represented by the general formula (IV) with carboxylic acids and for the production of nitroaromatic compounds by nitrating aromatic compounds with a nitrating agent, as previously explained.

For producing the hydroxyaryl aryl ketones, the same technique as in the case of using the aforementioned fluorine-containing sulfonated polyphenylsiloxane for the catalyst can be employed. Thus, using the same starting materials, the same hydroxyaryl aryl ketones can be produced under the same reaction conditions.

For producing the nitroaromatic compounds, the same technique as in the case of using the flruorine-containing sulfonated polyphenylsiloxane as the catalyst can be employed. Namely, the same aromatic compounds can be produced from the same starting materials under the same reaction conditions.

For producing the acyl-substituted aromatic compounds, the same technique as in the case of using the fluorine-containing sulfonated polyphenylsiloxane as the catalyst can be employed. Thus, the same acyl-substituted aromatic compounds can be produced using the same starting materials under the same reaction conditions.

⟨⟨Fluorine-containing Aromatic Sulfonic Acid/Clay Catalyst⟩⟩

Now, Fluorine-containing aromatic sulfonic acid/clay catalyst in which the matrix substance is a clay is explained.

As the clay capable of employing as the matrix substance, there may be enumerated, for example, cross-linking and non cross-linking bentonites, cross-linking and non cross-linking montmorillonites, cross-linking natural mica and cross-linking synthetic mica and so on. Among them, cross-linking montmorillonite, cross-linking natural mica and cross-linking synthetic mica are preferable.

For preparing the fluorine-containing aromatic sulfonic acid/clay catalyst, a method of introducing an aromatic group having substituents of fluoride and sulfonic acid group into the clay (corresponding to the production process 5 explained previously) may be employed. A concrete practice therefor may be as follows:

1 part by weight of a commercial clay, such as a montmorillonite or a synthetic mica, are colloidized in 50 - 500 parts by weight of water and the clay is subjected to an ion-exchange of the exchangeable ions therein by adding to the colloidized slurry an adequate amount of a solution containing ions for effecting cross-linking, such as aluminum chlorohydroxide complex. After completion of the ion-exchange, the clay is washed with water and is dried at a temperature of 50 - 500°C to obtain the contemplated cross-linking clay.

1 part by weight of the so obtained cross-linking caly and 1 - 10 parts by weight of an aromatic compound having substituents of fluoride and sulfonic acid group, such as chloromethylfluorobenzenesulfonic acid, are introduced into an adequate amount of an organic solvent and are caused to react in the presence of an organic or inorganic base at a temperature of 0 - 100°C, whereby a fluorine-containing aromatic sulfonic acid/clay catalyst in which the aromatic group having substituents of fluoride and sulfonic acid group is combined to the clay can be obtained.

The thus prepared fluorine-containing aromatic sulfonic acid/clay catalyst can be employed for the production of hydroxyaryl aryl ketones from phenol compounds expressed by the general formula (II) and aromatic carboxylic compounds represented by the general formula (III) through esterification and Fries rearrangement, for the production of acyl-substituted aromatic compounds by reacting the aromatic compounds represented by the general formula (IV) with carboxylic acids and for the production of

nitroaromatic compounds by nitrating aromatic compounds with a nitrating agent, as previously explained.

For producing the hydroxyaryl aryl ketones, the same technique as in the case of using the aforementioned fluorine-containing sulfonated polyphenylsiloxane for the catalyst can be employed. Thus, using the same starting materials, the same hydroxyaryl aryl ketones can be produced under the same reaction conditions.

For producing the nitroaromatic compounds, the same technique as in the case of using the fluorine-containing sulfonated polyphenylsiloxane as the catalyst can be employed. Namely, from the same starting materials, the same aromatic compounds can be produced under the same reaction conditions.

For producing the acyl-substituted aromatic compounds, the same technique as in the case of using the fluorine-containing sulfonated polyphenylsiloxane as the catalyst can be employed. Thus, the same acyl-substituted aromatic compounds can be produced using the same starting materials under the same reaction conditions.

## The Best Mode for Carrying out the Invention

Below, the present invention will be described by way of Examples.

### Example 1

72 g (0.30 mole) of phenyltriethoxysilane, 146 g (0.70 mole) of tetraethoxysilane, 120 ml of ethanol and 35 ml of 0.01 M solution of hydrogen chloride were charged in a 500 ml separable flask equipped with a distillation head and a stirrer and the mixture was heated at 100 °C with agitation to distill off 200 ml of ethanol over about 1 hour.

The residue was transferred to a 2 liter reactor made of SUS steel equipped with a stirrer together with 90 ml of cyclohexane and thereto was added about 1 liter of distilled water with vigorous agitation. Then, the mixture was agitated for 4 hours with addition of 100 ml of concentrated ammonia water.

After 4 hours, the solid matter formed was filtered, washed with water and dried under the condition of 150 °C, 20 mmHg and 10 hours, to obtain a polyphenylsiloxane in a form of white fine powder.

5 g of the so obtained polyphenylsiloxane were introduced into 50 g of carbon tetrachloride and thereto were added 2.5 g of xenon fluoride in order to cause reaction at room temperature for 3 hours, whereby a fluorine-containing polyphenylsiloxane was obtained. 20 g of the so obtained fluorine-containing polyphenylsiloxane and 200 ml of chloroform were charged in a two-necked 500 ml flask equipped with a reflux condenser and a stirrer, into which 50 ml of chlorosulfuric acid were dropped at room temperature under nitrogen atmosphere over 30 minutes. The mixture was then heated on an oil bath at 80 °C and was agitated for 4 hours.

Then, this reaction mixture was poured into 2 liters of ice water and the solid matter was filtered off, washed with water and dried under the condition of 150 °C, 20 mmHg and 10 hours, whereby a fluorine-containing sulfonated polyphenylsiloxane in a form of pale brown fine powder was obtained.

The content of $-SO_3H$ group in the so obtained product determined by ion-exchange technique was found to be 0.6 meq/g. The result of elementary analysis of this product was as follows:
Si = 29.5 wt-%, C = 6.58 wt-%, S = 3.34 wt-% and F = 0.38 wt-%

Fourier transform IR spectroscopy for this product gave the following data:

| Wave Number | Relative Strength | Resp. Chemical Bond |
|---|---|---|
| 1350 $cm^{-1}$ | Weak | S=O |
| 1440 $cm^{-1}$ | Weak | C-F |
| 1480 $cm^{-1}$ | Strong | Si- 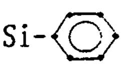 |
| 1510 $cm^{-1}$ | Strong | 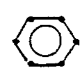 |
| 1600 $cm^{-1}$ | Strong | 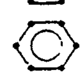 |

From the above, the proportion of each of the repeating units expressed by the general formulae (V-a)

to (V-d) in the so obtained fluorine-containing sulfonated polyphenylsiloxane is recognized to be 2:7:90:1 in the mole ratio of (V-a):(V-b):(V-c):(V-d).

Example 2

A sulfonation was carried out under the same conditions as in Example 1, except that instead of the fluorine-containing polyphenylsiloxane a polyphenylsiloxane was employed, whereby a sulfonated polyphenylsiloxane was obtained. Then, using this sulfonated polyphenylsiloxane, fluorination was carried out under the same conditions as in Example 1, whereby a fluorine-containing sulfonated polyphenylsiloxane was obtained. The result of elementary analysis of the so obtained product was as follows:
Si = 30.2 wt-%, C = 7.4 wt-%, S = 3.60 wt-% and F = 0.27 wt-%

Example 3

0.35 g of the fluorine-containing sulfonated polyphenylsiloxane catalyst obtained in Example 1, 1.72 g (0.019 mole) of hydroxybenzene (namely, phenol) and 0.48 g (0.004 mole) of benzoic acid were charged in a reaction vessel and benzoylation of hydroxybenzene was carried out. The analysis of the composition of the so obtained product by gas chromatography showed that the conversion of benzoic acid was found to be 100 % and the selectivity of hydroxybenzophenone was determined to be 62 % (based on benzoic acid) , with a phenyl benzoate formation of 36 % (based on benzoic acid).

Comparative Example 1

Benzoylation of hydroxybenzene was conducted under the same condition as in Example 3, except that the fluorine-containing sulfonated polyphenylsiloxane was changed to a sulfonated polyphenylsiloxane. The conversion of benzoic acid was found to be 86 % and the selectivity of hydroxybenzophenone was determined to be 44 % (based on benzoic acid) with a phenol benzoate formation of 55 % (based on benzoic acid).

Example 4

Benzoylation of hydroquinone was carried out under the same condition as in Example 3, except that the phenol employed was changed to hydroquinone. The conversion of benzoic acid was found to be 100 % and the selectivity of dihydroxybenzophenone was determined to be 38 % (based on benzoic acid), besides 51 % (based on benzoic acid) of benzoic acid p-hydroxyphenyl ester had been formed.

Comparative Example 2

Benzoylation of hydroquinone was carried out under the same condition as in Example 4, except that the fluorine-containing sulfonated polyphenylsiloxane was changed to a sulfonated polyphenylsiloxane. The conversion of benzoic acid was found to be 92 % and the selectivity of dihydroxybenzophenone was determined to be 21 % (based on benzoic acid), besides 68 % (based on benzoic acid) of benzoic acid p-hydroxyphenyl ester had been formed.

Example 5

1 g of the fluorine-containing sulfonated polyphenylsiloxane obtained in Example 1 was charged in a quartz reaction tube and was heated, while flowing $N_2$ gas, to a temperature of 180 °C . Then, nitration of benzene was carried out, by supplying to this reaction tube benzene at a flow rate of 4 ml/hr and $NO_2$ gas at a flow rate of 20 ml/min. Nitrobenzene was obtained at a yield of 82 %.

Comparative Example 3

Nitration of benzene was carried out under the same condition as in Example 5, except that the fluorine-containing sulfonated polyphenylsiloxane was changed to a sulfonated polyphenylsiloxane. The yield of nitrobenzene was found to be 65 %.

Example 6

8.0 g of chlorotriethoxysilane were dissolved in 100 ml of ether and the solution was agitated on a dry ice/acetone bath. Thereto were dropped 20 ml of 2.0 M ether solution of p-fluorophenyl magnesium bromide

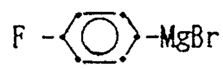

to cause the reaction. The pale yellow oily product remaining after distilling off ether was dissolved in 100 ml of ethanol and thereto were added 20 g of tetraethoxysilane and 35 ml of 0.1 N hydrochloric acid. The mixture was heated at 120 °C while agitation for 2 hours in order to distill off the ethanol.

To the so obtained residue, 100 ml of cyclohexane and 60 ml of ethanol were added. This mixture was poured into 500 ml of distilled water with vigorous agitation and thereto were then added 100 ml of ammonia water.

After 8 hours, the solid matter formed was filtered off, washed with water and dried at 100 °C for 8 hours, whereby 14 g of fluorine-containing polyphenylsiloxane in a form of white powder were obtained.

13 g of the so obtained pulverous product were introduced into 50 ml of chloroform and thereto were poured 100 ml of a liquid mixture of chloroform and $ClSO_3H$ in a proportion of 5:1 (volume ratio) to effect sulfonation. After filtration, water wash and drying at 100°C, 11 g of the objective fluorine-containing sulfonated polyphenylsiloxane catalyst were obtained. By the elementary analysis, the composition of this fluorine-containing sulfonated polyphenylsiloxane was found to be:
F = 2.6 wt-%, S = 3.5 wt-%, C = 12.0 wt-% and Si = 33.0 wt-%

The proportion of each of the repeating units expressed by the general formulae (V-a) to (V-d) in the so obtained fluorine-containing sulfonated polyphenylsiloxane is recognized to be 12:85:3 in the mole ratio of (V-a): (V-c): (V-d). By the way, it is recognizable that the content of repeating unit (V-b) is quite small.

Then, water-repellent property of the thus obtained product was estimated using a thermobalance. About 30 mg of dried fluorine-containing sulfonated polyphenylsiloxane catalyst were accurately weighed. Moisture was caused to be adsorbed on this dried catalyst up to saturation by flowing nitrogen gas containing about 3 volume % of moisture at room temperature.

Then, the stream of nitrogen gas was changed over to that of dry nitrogen gas without containing moisture so as to cause desorption of moisture. The initial moisture desorption rate observed was used as the measure of water repellency. Further, the strength of acidity ($H_o$) of the catalyst was determined by a usual indicator method. Results are recited in Table 1.

Comparative Examples 4 and 5

A sulfonated polyphenylsiloxane without fluorine content (Comparative Example 4) and a silica-alumina product (Comparative Example 5) were tested for the initial moisture desorption rate and for the strength of acidity ($H_o$) in the same manner as in Example 6. Results are summarized also in Table 1.

Table 1

| Example | Product | Initial Rate of $H_2O$ Desorption (g/min·acid site) | Acid Strength ($H_o$) |
|---|---|---|---|
| 6 | F-SPS [1)] | 210 | -11.4 |
| 4 Compar. | SPS [2)] | 18 | - 3.0 |
| 5 Compar. | Silica-Alumina | 1 | - 5.6 |

Notes: 1): F-SPS: Fluorine-containing sulfonated polyphenylsiloxane obtained in Example 6

2): SPS : Sulfonated polyphenylsiloxane

Example 7

0.2 g of the fluorine-containing sulfonated polyphenylsiloxane obtained in Example 6, 20 ml of mesitylene (i.e. 1,3,5-trimethylbenzene) and 0.5 g of caprylic acid were charged in a 50 ml flask and acylation of mesitylene was carried out at 180 °C for 5 hours. Results are recited in Table 2.

Comparative Examples 6 and 7

Acylation of mesitylene was carried out under the same condition as in Example 6, except that the catalyst was replaced by Nafion-H (trademark; a product of E.I. du Pont de Nemours and Co. - Comparative Example 6) by a silica-alumina product (Comparative Example 7). Results are also given in Table 2.

Comparative Example 8

5 g of a commercial ion-exchange resin of sulfonic acid type (Amberlyst 15 (trademark) of Rohm and Haas Co. ) were introduced into 50 g of carbon tetrachloride and thereto were added 2.5 g of xenon fluoride to effect fluorination at room temperature for 3 hours, whereby a product of fluorine-containing Amberlyst 15 was obtained.

Using this product as the catalyst, the reaction was carried out as in Example 7. Results are also given in Table 2.

Table 2

| Example | Catalyst | Conversion of Caprylic Acid (%) |
|---|---|---|
| 7 | F-SPS [1)] | 45 |
| 6 Compar. | Nafion-H | 5 |
| 7 Compar. | Silica-Alumina | 0 |
| 8 Compar. | F-A15 [2)] | 21 |

Notes: 1): F-SPS: Fluorine-containing sulfonated polyphenylsiloxane obtained in Example 6
2): F-A15: Fluorine-containing Amberlyst 15

Example 8

0.2 g of the fluorine-containing sulfonated polyphenylsiloxane obtained in Example 6, 20 ml of m-xylene and 15.6 g of benzoic anhydride were charged in a reaction vessel and acylation of m-xylene was carried out under reflux of m-xylene for 2 hours. Results are recited in Table 3.

Examples 9 and 10

Acylation was carried out under the same conditions as in Example 8, except that m-xylene was replaced by toluene (Example 9) or by anisole (Example 10). Results are also given in Table 3.

Examples 11 to 13

Acylation of m-xylene was carried out under the same condition as in Example 8, except that benzoic anhydride was replaced by benzoic acid (Example 11), by propionic acid (Example 12) or by acetic acid (Example 13). Results are also given in Table 3.

Comparative Examples 9 to 11

Acylation of m-xylene was carried out under the same condition as in Example 8, except that the fluorine-containing sulfonated polyphenylsiloxane catalyst was replaced by a sulfonated polyphenylsiloxane without containing fluorine (Comparative Example 9), by p-toluenesulfonic acid (Comparative Example 10) or by silica-alumina (Comparative Example 11). Results are also given in Table 3.

Table 3

| Example | Catalyst | Conversion of Carboxylic Acid (%) | Selectivity of Acylated Product (%) |
|---|---|---|---|
| 8 | F-SPS 1) | 96 | 98 |
| 9 | F-SPS | 65 | 97 |
| 10 | F-SPS | 82 | 99 |
| 11 | F-SPS | 86 | 97 |
| 12 | F-SPS | 50 | 98 |
| 13 | F-SPS | 28 | 96 |
| 9 Compar. | SPS 2) | 46 | 90 |
| 10 Compar. | p-TSA 3) | 34 | 91 |
| 11 Compar. | Silica-Alumina | 10 | 87 |

Notes: 1): F-SPS: Fluorine-containing sulfonated polyphenylsiloxane obtained in Example 6
2): SPS : Sulfonated polyphenylsiloxane
3): p-TSA : p-Toluenesulfonic acid

Example 14

A catalyst was prepared under the same condition as in Example 6, except that p-fluorophenyl magnesium bromide employed in Example 6 was replaced by 3,5-bis(trifluoromethyl)phenyl magnesium bromide:

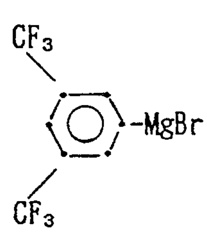

The so obtained fluorine-containing sulfonated polyphenylsiloxane had a composition:
F = 10.2 wt-%, S = 3.1 wt-%, C = 13.2 wt-% and Si = 34.6 wt-%

Then, acylation of mesitylene was carried out under the same condition as in Example 7, except that the catalyst was replaced by the above fluorine-containing sulfonated polyphenylsiloxane. A conversion of caprylic acid was founf to be 38 %.

Example 15

To a 1 M aqueous solution of phosphoric acid, a 0.5 M aqueous solution of zirconium oxychloride was added and the reaction was effected at about 0° C overnight. After filtration, the product was reslurried using an aqueous solution of phosphoric acid of a concentration of 5 % by weight and this mixture was conditioned at 40° C for 1 hour. After washing with water, the product was dried at 100° C for 15 hours and the so dried product was added to a 16 M aqueous phosphoric acid and the mixture was heated at about 165 ° C for 10 hours, whereby a crystalline zirconium phosphate was obtained.

5 g of the so obtained zirconium phosphate were caused to react with 20 g of chloromethylfluorobenzenesulfonic acid in the presence of a base, whereby a catalyst comprising zirconium phosphate, into which the fluorine-containing benzenesulfonic acid groups had been introduced, was obtained.

0.2 g of the above catalyst, 20 ml of mesitylene and 0.5 g of caprylic acid were charged in a 50 ml flask and acylation of mesitylene was carried out at 180° C for 5 hours. The conversion of caprylic acid was found to be 35 %.

Comparative Example 12

Acylation of mesitylene was carried out under the same condition as in Example 15, except that the catalyst was replaced by zirconium phosphate.. The conversion of caprylic acid was found to be 0 %.

Example 16

To 1 M aqueous solution of aluminum chloride, 200 g of aluminum were added gradually and the reaction was conducted at 50 ° C for 6 hours. After filtering off the unreacted aluminum, an aqueous solution containing aluminum chlorohydroxide complex was obtained. The amount of aluminum in this aqueous solution was determined to be 10.0 % by weight.

100 g of 10 wt. % aqueous suspension of a synthetic mica, i.e. sodium tetrasilicic mica [$NaMg_{2.5}(Si_4O_{10})F_2$], a product of Topie Kogyo K.K.) was poured into 1 liter of water and the mixture was agitated well. To this suspension, 300 g of an aqueous solution containing the above aluminum chlorohydroxide complex were added gradually with agitation to cause ion-exchange. After filtration, water wash and drying at 120 ° C overnight, a cross-linking synthetic mica was obtained.

5 g of this cross-linking synthetic mica were reacted with 20 g of chloromethylfluorobenzenesulfonic aicd in the presence of a base, whereby a catalyst comprising a cross-linking synthetic mica, into which fluorine-containing benzenesulfonic acid groups had been introduced, was obtained.

0.2 g of the above catalyst, 20 ml of mesitylene and 0.5 g of caprylic acid were charged in a 50 ml flask and acylation of mesitylene was carried out at 180° C for 5 hours. The conversion of caprylic acid was found to be 41 %.

Comparative Example 13

Acylation of mesitylene was carried out under the same condition as in Example 16, except that the catalyst was replaced by cross-linking synthetic mica. The conversion of caprylic acid was found to be 0%.

Example 17

A catalyst was prepared in the same manner as in Example 16, except that 100 g of 10 wt-% aqueous suspension of sodium tetrasilicic mica was replaced by 10 g of a pulverous montmorillonite, and acylation of mesitylenen was carried out. The conversion of caprylic acid was found to be 33 %.

Comparative Example 14

Acylation of mesitylene was carried out under the same condition as in Example 17, except that the catalyst was replaced by oross-linking montmorillonite. The conversion of caprylic acid was found to be 1

%.

Applicability in Industry

while the fluorine-containing aromatic sulfonic acid catalyst produced according to the present invention can be utilized suitably as a catalyst for acylation and nitration, it can be utilized also for various acid catalysis reaction, such as, esterification, alkylation, etherification and so on.

## Claims

1. A fluorine-containing aromatic sulfonic acid catalyst comprising a matrix substance selected from the group consisting of polysiloxanes and inorganic substances, to which are combined substituted aryl groups represented by the general formula (I),

$$(X)_n-Ar^1-(SO_3H)_m \quad \cdots\cdots \quad (I)$$

wherein X denotes fluorine atom or a fluorine-containing $C_1$ - $C_{14}$ alkyl group which may or may not contain etheric oxygen atom, $Ar^1$ represents an aryl group, n is an integer of 1 - 5, with X being the same or different with each other for the case of n ≧ 2, and m is an integer of 1 - 3.

2. A fluorine-containing aromatic sulfonic acid catalyst as claimed in Claim 1, wherein the matrix substance is a polysiloxane and $Ar^1$ in the general formula (I) is phenyl group.

3. A fluorine-containing aromatic sulfonic acid catalyst as claimed in Claim 1, wherein the matrix substance is a zirconium compound.

4. A fluorine-containing aromatic sulfonic acid catalyst as claimed in Claim 1, wherein the matrix substance is a clay.

5. A fluorine-containing aromatic sulfonic acid catalyst as claimed in Claim 4, wherein the matrix substance is a cross-linking mica or a cross-linking montmorillonite.

6. A process for the production of a fluorine-containing aromatic sulfonic acid catalyst comprising a polysiloxane to which are combined substituted aryl groups represented by the general formula (I-a),

$$(X)_n-Ar^1-(SO_3H)_m \quad \cdots\cdots \quad (I\text{-}a)$$

wherein X denotes fluorine atom or a fluorine-containing $C_1$ - $C_{14}$ alkyl group which may or may not contain etheric oxygen atom, $Ar^1$ represents phenyl group, n is an integer of 1 - 5, with X being the same or different with each other for the case of n ≧ 2, and m is an integer of 1 - 3, said process comprising subjecting a polyphenylsiloxane to fluorination and to sulfonation.

7. A process for the production of a fluorine-containing aromatic sulfonic acid catalyst comprising a polysiloxane to which are combined substituted aryl groups represented by the general formula (I-b),

$$(X)_n-Ar^1-(SO_3H)_m \quad \cdots\cdots \quad (I\text{-}b)$$

wherein X denotes fluorine atom or a fluorine-containing $C_1$ - $C_{14}$ alkyl group which may or may not contain etheric oxygen atom, $Ar^1$ represents phenyl group, n is an integer of 1 - 5, with X being the same or different with each other for the case of n ≧ 2, and m is an integer of 1 - 3, said process

comprising subjecting a fluorine-containing polyphenylsiloxane to sulfonation with a sulfonating agent.

**8.** A process for the production of a fluorine-containing aromatic sulfonic acid catalyst comprising a polysiloxane to which are combined substituted aryl groups represented by the general formula (I-c),

$$(X)_n-\underset{|}{Ar^1}-(SO_3H)_m \quad \cdots\cdot \quad (I\text{-}c)$$

wherein X denotes fluorine atom or a fluorine-containing $C_1$ - $C_{14}$ alkyl group which may or may not contain etheric oxygen atom, $Ar^1$ represents phenyl group, n is an integer of 1 - 5, with X being the same or different with each other for the case of $n \geqq 2$, and m is an integer of 1 - 3, said process comprising subjecting a polyphenylsiloxane having sulfonic acid groups to fluorination with a fluorine-containing compound.

**9.** A process for the production of a fluorine-containing aromatic sulfonic acid catalyst comprising a zirconium compound to which are combined substituted aryl groups represented by the general formula (I-d),

$$(X)_n-\underset{|}{Ar^1}-(SO_3H)_m \quad \cdots\cdot \quad (I\text{-}d)$$

wherein X denotes fluorine atom or a fluorine-containing $C_1$ - $C_{14}$ alkyl group which may or may not contain etheric oxygen atom, $Ar^1$ represents an aryl group, n is an integer of 1 - 5, with X being the same or different with each other for the case of $n \geqq 2$, and m is an integer of 1 - 3, said process comprising subjecting a zirconium compound to a reaction with a compound of fluorine-containing aromatic sulfonic acid.

**10.** A process for the production of a fluorine-containing aromatic sulfonic acid catalyst comprising a clay to which are combined substituted aryl groups represented by the general formula (I-e),

$$(X)_n-\underset{|}{Ar^1}-(SO_3H)_m \quad \cdots\cdot \quad (I\text{-}e)$$

wherein X denotes fluorine atom or a fluorine-containing $C_1$ - $C_{14}$ alkyl group which may or may not contain etheric oxygen atom, $Ar^1$ represents an aryl group, n is an integer of 1 - 5, with X being the same or different with each other for the case of $n \geqq 2$, and m is an integer of 1 - 3, said process comprising subjecting a clay to a reaction with a compound of fluorine-containing aromatic sulfonic acid.

**11.** A process for the production of a hydroxyaryl aryl ketone by reacting a phenol represented by the general formula (II),

$$Ar^2-(OH)_m \quad \cdots\cdot \quad (II)$$

with an aromatic carboxylic acid represented by the general formula (III),

$$Ar^3-(COOH)_n \quad \cdots\cdot \quad (III)$$

wherein $Ar^2$ and $Ar^3$ denotes each an aryl group and n and m represent each an integer of at least 1, said process comprising conducting the reaction using a fluorine-containing aromatic sulfonic acid catalyst as claimed in Claim 1.

**12.** A process for the production of an acyl-substituted aromatic compound by reacting a carboxylic acid with an aromatic compound represented by the general formula (IV),

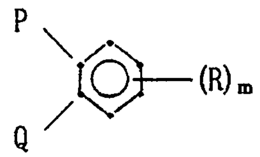

.... (IV)

wherein R represents hydrogen atom, an alkyl group, an alkoxy group or a halogen atom, m is an integer of 1 - 3 and P and Q denote both hydrogen atom or either of them is phenyl group and the other is hydrogen atom or they represent each a hydrocarbon group combining with each other to build up a substituted or unsubstituted naphthalene or tetralin ring, said process comprising conduoting the reaction using a fluorine-containing aromatic sulfonic acid catalyst as claimed in Claim 1.

**13.** A process for the production of an aromaric nitro compound by reacting an aromatic compound with a nitrating agent, said process comprising conducting the reaction using a fluorine-containing aromatic sulfonic acid catalyst as claimed in Claim 1.

# INTERNATIONAL SEARCH REPORT

International Application No PCT/JP89/00666

**I. CLASSIFICATION OF SUBJECT MATTER** (if several classification symbols apply, indicate all) [6]

According to International Patent Classification (IPC) or to both National Classification and IPC

Int. Cl$^4$ B01J31/02, 31/10, C07C45/45, 49/76, 76/02, 79/10, C08G77/24, 77/28, 77/38

**II. FIELDS SEARCHED**

Minimum Documentation Searched [7]

| Classification System | Classification Symbols |
|---|---|
| IPC | B01J31/02, 31/10, C07C45/45, 49/76-49/83, 76/02, 79/10, C08G77/24, 77/28, 77/38 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, B2, 60-25190 (Shell Internationale Research Maatschappy N. V.) 17 June 1985 (17. 06. 85) & JP, A, 52-106391 & US, A, 4038213 & DE, A1, 2709057 & NL, A, 7702211 & FR, A1, 2342786 & GB, A, 1564272 | 1 - 5 |
| A | JP, A, 61-282335 (Honshu Chemical Industry Co., Ltd.) 12 December 1986 (12. 12. 86) (Family: none) | 11 |
| A | JP, A, 50-154212 (Mitsubishi Chemical Industries Ltd.) 12 December 1975 (12. 12. 75) (Family: none) | 13 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| September 4, 1989 (04. 09. 89) | September 18, 1989 (18. 09. 89) |
| **International Searching Authority** | **Signature of Authorized Officer** |
| Japanese Patent Office | |